# EUROPEAN PATENT SPECIFICATION

(11) **EP 2 874 992 B1**
(45) Date of publication and mention of the grant of the patent: **15.04.2020**
(21) Application number: 13740349.9
(22) Date of filing: 18.07.2013
(51) Int. Cl.: C07C 67/343, C07C 253/30, C01B 21/092, C07C 69/63, C07C 255/21

(54) **PROCESS FOR CROSSED CLAISEN CONDENSATION REACTIONS PROMOTED BY LITHIUM AMIDE IN LIQUID AMMONIA**
VERFAHREN FÜR DURCH LITHIUMAMID IN FLÜSSIGEM AMMONIA GEFÖRDERTE CLAISEN-KREUZKONDENSATIONREAKTIONEN
PROCÉDÉ POUR DES RÉACTIONS DE CONDENSATION DE CLAISEN CROISÉES FAVORISÉES PAR L'AMIDURE DE LITHIUM DANS L'AMMONIAC LIQUIDE

(30) Priority: 18.07.2012 GB 201212777
(43) Date of publication of application: 27.05.2015
(73) Proprietor: Bakhu Limited, Southport PR8 2EY (GB)
(72) Inventor: LEECE, Colin, Bromborough Wirral CH62 3PW (GB); PROCTOR, Lee David, Nercwys Flintshire CH7 4AU (GB)
(74) Representative: TL Brand & Co
(86) International application number: PCT/GB2013/051926
(87) International publication number: WO 2014/013261

(56) References cited:
- WO-A1-2011/048425
- WO-A2-2005/080265
- US-B2- 6 903 225

## Description

### Field of the invention

The present invention relates to a method of carrying out a crossed Claisen condensation reaction using lithium amide in liquid ammonia. More specifically, the present invention relates to the use of lithium amide in liquid ammonia in a crossed Claisen condensation reaction involving TBA and HN or ECHB.

### Background

Crossed Claisen condensation reactions are commonly known in the art as reactions that occur between two esters, or between one ester and another carbonyl compound, in the presence of a strong base to produce an enolate from at least one ester, resulting in the production of a β-keto ester or a β-diketone.

Crossed Claisen condensation reactions can be used to produce useful products such as BHA or ATS-8 by reacting tert-butyl acetate (TBA) with ethyl (S)-4-chloro-3-hydroxybutanoate (ECHB) or (R)-ethyl-4-cyano-3-hydroxybutyrate (HN).

However, crossed Claisen condensation reactions can result in a total of four different keto-ester products if the two esters used in the reaction are both enolizable. This reduces the synthetic utility of the reaction and consequently crossed Claisen condensation reactions are classically reserved for reactions in which only one of the ester substrates contains enolizable hydrogen atoms, because in this case only two of the four keto-ester products can be formed.

Strong, hindered, non-nucleophilic lithium amides, such as lithium diisopropylamide (LDA) and lithium hexamethyldisilazide (LHMDS), are generally used as the base to produce the enolate required for the crossed Claisen condensation reaction. These bases are chosen because they are strong bases (pKa 26-36) that rapidly de-protonate the substrate ester (in this case TBA) to quantitatively form the lithium enolate. Further, hindered bases contain bulky alkyl or silyl-alkyl groups, which are deemed important to prevent the base reacting to produce unwanted by-products, such as homologated amides.

For example, EP-A-0643689 discloses the treatment of tertiary butyl acetate with a strong base such as lithium diisopropylamide, so as to produce a product for use in a crossed Claisen condensation reaction.

Further, US 6,340,767 discloses the use of a lithium amide base of formula (III) in a crossed Claisen condensation reaction, wherein the R groups of formula (III) are alkyl groups of 1 to 12 carbon atoms, aryl groups of 6 to 12 carbon atoms, aralkyl groups of 7 to 12 carbon atoms and/or silyl groups.

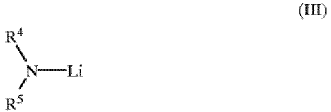

However, the use of these hindered, non-nucleophilic bases has various disadvantages. First, they are typically prepared from the corresponding amines by metal exchange. For example, LDA is prepared before use by reacting diisopropylamine with highly pyrophoric alkyl-lithium bases such as n-butyl lithium or n-hexyl lithium. LHMDS can be prepared before use by reacting hexamethyldisilazane with lithium amide under reflux conditions in ethereal solvents. The use of n-butyl or n-hexyl lithium also requires potentially unwanted solvents such as hexane. Further, n-butyl lithium generates gaseous butane as a by-product, which has environmental implications.

Further, when the reaction is quenched, for example with aqueous hydrochloric acid, diisopropylamine is converted into its hydrochloride salt and hexamethyldisilazane is hydrolysed to hexamethyldisiloxane. This makes base recovery very difficult and costly to such a degree that in normal practice the bases are not recovered. This results in higher waste burdens and processing costs.

The use of lithium amide as a base was first pioneered by Charles Hauser in the 1950s and 1960s. In J. Am. Chem. Soc. 1953, 75, 1068-1072, Hauser describes the use of lithium amide in liquid ammonia for producing lithium TBA-enolate and its use in the Aldol condensation with a variety of ketone and aldehyde substrates. In J. Org. Chem. 1960, 25, 503-507, Hauser expands the scope of his original work to include the reaction of the lithium enolate of ethylacetate with a variety of ketone and aldehyde substrates.

US 6,903,225 discloses the difficulties in preventing the self Claisen condensation reaction, so as to improve the yield of the more useful product of the crossed Claisen condensation reaction. Step 1 of US 6,903,225 involves reacting a structure that covers tert-butyl acetate enolate with a structure that covers ECHB. Lithium amide is disclosed among a longer list of more hindered bases. However, preferred bases are disclosed as having the formula (X), which is more hindered than lithium amide and all of the examples use lithium diisopropylamide.

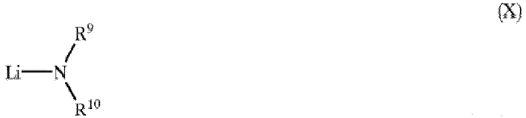

WO2011/048425 discloses lithium amide as a base for use in a crossed Claisen condensation reactior between a structure that covers tert-butyl acetate enolate and a structure that covers both ECHB and HN, as part of a longer list including more hindered bases. The process described is a continuous process with reaction times of less than five minutes.

However, it has been found that lithium amide alone does not function well as a base in the reactions disclosed in these prior art documents, possibly due to its low solubility. This is demonstrated in comparative example A of WO2005/080265 (EP1716078), which attempts the reaction of lithium amide with TBA at -40°C and 20°C in the absence of liquid ammonia. In both cases, no reaction was observed. WO2005/080265 (EP1716078) does disclose the use of lithium amide in liquid ammonia for a self Claisen ester condensation reaction.

However, lithium amide in liquid ammonia was not thought to be suitable for use in crossed Claisen condensation reactions, especially those in which one or more of the esters contain a hydroxyl group. The lithium amide in liquid ammonia was thought to initially de-protonate the alcohol group to form a lithium alkoxide. The lithium metal of this alkoxide is ideally situated to coordinate to a neighbouring ester group and this coordination enhances the reactivity of the ester towards nucleophilic displacement.

If the reactivity of the ester is enhanced towards nucleophilic attack it would be expected that any other nucleophile present would compete with the desired nucleophilic displacement by the enolate, thereby resulting in amide impurities. It is for this reason that most of the prior art processes for the crossed Claisen condensation reaction between HN or ECHB and TBA use hindered non-nucleophilic bases such as LHMDS and LDA.

The use of lithium amide in liquid ammonia was also specifically avoided as the reagent system for carrying out Claisen condensation reactions with HN or ECHB (as discussed above) as these substrates were thought to react with either liquid ammonia or lithium amide in an unwanted fashion. For example, ECHB is an aliphatic alkyl chloride and it is known that such compounds undergo solvolysis with liquid ammonia according to pseudo first order kinetics with typical half-lives of 10-12 minutes (see Penju Ji et al., Liquid Ammonia as a Dipolar Aprotic Solvent for Aliphatic Nucleophilic Substitution Reactions, J. Org. Chem. Vol. 76, No. 5, 2011, pp1425-1435), as shown below.

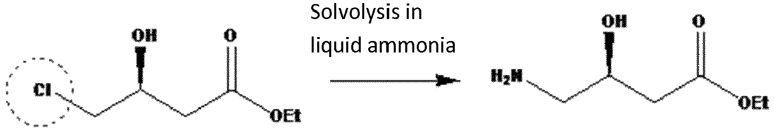

Similarly, the substrate HN contains a nitrile group which is both reactive and renders the neighbouring alpha-hydrogens acidic and enolizable, as shown below.

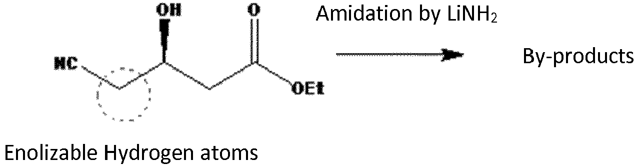

The present invention therefore relates to a novel method of carrying out a crossed Claisen condensation reaction, which overcomes the disadvantages of the crossed Claisen condensation reactions of the prior art, using lithium amide in liquid ammonia.

### Summary of the invention

As discussed above, it was previously known that the use of lithium amide as a base in crossed Claisen condensation reactions did not result in the desired products. However, the inventors surprisingly found that the use of lithium amide in liquid ammonia did result in the desired products and also overcame the disadvantages of the processes of the prior art.

Lithium amide is classed as a nucleophilic and un-hindered amide base. As such, it is fundamentally different from the preferred bases used in the prior art. The use of lithium amide in liquid ammonia gives clean products in a surprisingly high yield. No evidence of competing substitution or amide formation resulting from unwanted reactions with lithium amide has been observed.

This can be demonstrated by comparing the molar yields resulting from the method of the present invention to those of the prior art. For example, Example 8 of US6340767 discloses a highest yield of 57%, while WO2011/048425 discloses a highest yield of 72%. In contrast, the highest yield obtained with the method of the present invention was 92%. This improved performance suggests that the process of the present invention increases the selectivity of the desired crossed Claisen condensation reaction over potentially competing unwanted side reactions.

The use of strong, hindered, non-nucleophilic bases can be avoided completely by using lithium amide in liquid ammonia. This therefore overcomes the disadvantages of using these bases, such as the problems of making them from the corresponding amines by metal exchange and the increased difficulty and cost of base recovery.

Further, lithium amide itself has surprisingly been found to be better for use in the crossed Claisen condensation reaction than more conventional bases such as LHMDS, as fewer unwanted reaction products are formed.

A first aspect of the present invention therefore relates to the use of lithium amide in liquid ammonia as a base to produce an enolate from at least one ester starting material in a crossed Claisen condensation reaction, wherein at least one ester starting material is a β-hydroxy ester. The use is particularly beneficial when both ester starting materials for the crossed Claisen condensation reaction are enolizable. The use of lithium amide in liquid ammonia is thought to help minimise the self Claisen condensation reaction product.

Without wishing to be bound by theory, it is thought that lithium amide in liquid ammonia reacts with the alcohol group on the β-hydroxy ester to form a lithium alkoxide. The lithium metal of this alkoxide coordinates the neighbouring ester group and this coordination inhibits enolisation. This means that the β-hydroxy ester does not form an enolate, thereby preventing two of the four possible reactions in a crossed Claisen condensation reaction where both esters are enolisable. The use of lithium amide in liquid ammonia therefore allows crossed Claisen condensation reactions to be commercially viable, as two of the unwanted products will not be produced.

In an embodiment of the present invention, the lithium amide in liquid ammonia is produced *in situ.* This may be done by reacting lithium metal with liquid ammonia and an electron transfer agent. The electron transfer agents are preferably conjugated dienes, such as styrene or isoprene. However, any other suitable electron transfer agent known by the skilled person from the prior art, such as transition metals or salts thereof (e.g. Cobalt or Iron (III) nitrate) may be used. The lithium amide produced is sparingly soluble in liquid ammonia and this solubility is sufficient to facilitate the desired chemistry. In the absence of liquid ammonia the chemistry does not work (see Comparative Example 1).

A co-solvent is also preferably used during the reaction. The co-solvent is preferably an ethereal solvent, a hydrocarbon solvent or a hindered ethereal solvent. The co-solvent can be selected from tert-butyl methyl ether (TBME), THF or hexane.

A further advantage of using lithium amide in liquid ammonia is that once the crossed Claisen condensation reaction is complete the crude unquenched product mixture can be heated to drive off ammonia. The resulting ammonia gas can be recompressed and then recycled and/or re-used in the process. The product residue can be quenched using either aqueous mineral acids (for example hydrochloric acid or sulphuric acid) or carboxylic acids (for example acetic acid) to furnish the desired beta-keto ester products.

Further, it has been discovered that in certain co-solvents, for example TBME, the unquenched lithium salt of the product is insoluble, thereby facilitating its isolation and purification by filtration. A further advantage of the current process is that the crude unquenched product mixture can be quenched under anhydrous conditions using acidic ammonium salts such as ammonium chloride or ammonium nitrate. After such quenching the reaction mixture can be warmed and the resulting ammonia gas recompressed for re-use if desired.

The method of the present invention therefore provides efficient utilisation of chemicals. When implementing the invention, as shown in the following examples, a total of eight chemicals are used, three of which (ammonia, TBME and hexane) are easily recoverable. In contrast, the prior art documents disclose much less efficient systems. For example, Example 3 of US6903225 uses eleven chemicals, none of which are easily recoverable as diisopropylamine is converted into its hydrochloride salt and the distillate recovered is a mix of THF, hexane, toluene and ethyl acetate, which would be difficult to separate. WO2011/048425 discloses a method that, when using methods of producing lithium hexamethyldisilazane as described in the prior art, requires eight chemicals, two of which (THF and dichloromethane) are potentially separable after fractional distillation. The present invention therefore limits the waste produced by the process and optimises the process conditions.

In a further embodiment of the present invention, one of the ester starting materials is tert-butyl acetate. In this embodiment, the lithium amide in liquid ammonia is used to produce the tert-butyl acetate enolate.

This enolate can then be used in a crossed Claisen condensation reaction with a β-hydroxy ester. Preferably, this β-hydroxy ester is selected from HN or ECHB, as shown below. When X is CI, the compound is ECHB and when X is CN, the compound is HN.

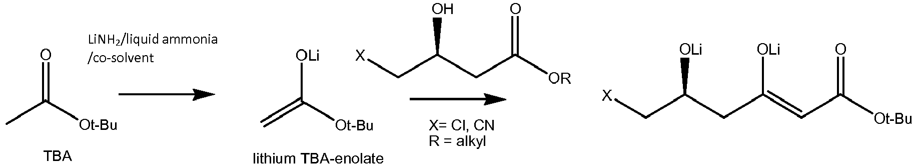

The overall reaction of this embodiment after quenching with an aqueous mineral acid, a carboxylic acid or an ammonium salt, so as to form ATS-8 or BHA for HN and ECHB respectively, is shown below.

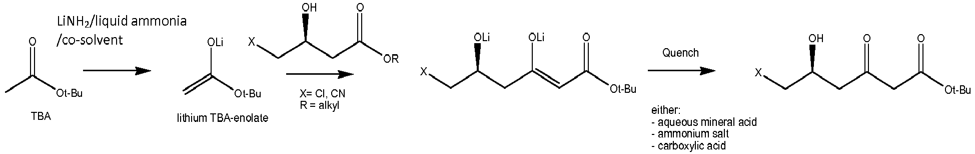

Alternatively, the tert-butyl acetate enolate may then be used in a crossed Claisen condensation reaction with the β-hydroxy ester (S)-3-hydroxy-butyrolactone, as shown below.

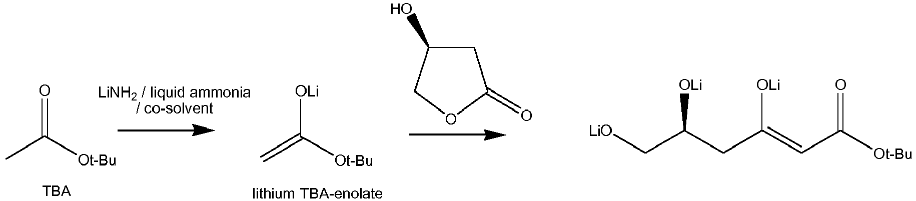

The overall reaction of this embodiment after quenching with an aqueous mineral acid, a carboxylic acid or an ammonium salt, so as to form (S)-tert-butyl 5,6-dihydroxy-3-oxohexanoate, is shown below.

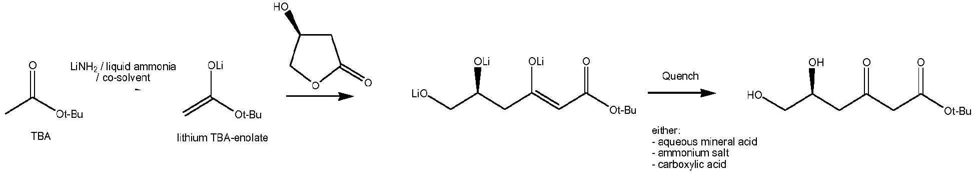

Also described herein is a method of producing lithium amide *in situ* for use in a crossed Claisen condensation reaction wherein lithium is added to liquid ammonia, followed by an electron transfer agent. Preferably, the reaction temperature at ambient pressure is maintained between -33 and -40°C, between -40 and -50°C, between -50 and -60°C or between -60 and -70°C. Preferably, the temperature at ambient pressure is maintained between -40 and -50°C. It has been found that reducing the temperature improves the selectivity of the reaction but reduces the reaction rate.

The method may also be carried out at an increased pressure. The pressure and temperature must be balanced such that the ammonia exists in its liquid state. The table below illustrates the reaction temperatures possible at increased pressures. Any of the temperature/pressure combinations may be selected.

| Pressure (bar) | Temperature (°C) |
|---|---|
| 1.196 | -30 |
| 1.902 | -20 |
| 2.908 | -10 |
| 4.295 | 0 |
| 6.149 | 10 |
| 8.570 | 20 |
| 11.67 | 30 |
| 15.54 | 40 |
| 20.33 | 50 |

In another aspect, the present invention provides a method of carrying out a crossed Claisen condensation reaction using an ester starting material and a β-hydroxy ester, comprising the step of producing lithium amide *in situ,* as described above, wherein lithium is added to liquid ammonia, followed by an electron transfer agent, wherein: i) lithium is added in a stoichiometric ratio with respect to the β-hydroxy ester of 3.0 to 5.0; and/or ii) ammonia is added in a stoichiometric ratio with respect to β-hydroxy ester of 5 to 30.

The method may involve adding lithium in a stoichiometric ratio with respect to the β-hydroxy ester of 3.0 to 5.0, or any range of 0.5 therebetween. Preferably the ratio is between 3.5 and 4.0, more preferably 3.75.

The method may involve adding ammonia in a stoichiometric ratio with respect to the β-hydroxy ester of 5 to 30, or any range of 5 therebetween. Preferably the ratio is between 20 and 25, more preferably 21.

The method may involve adding an ester starting material in a stoichiometric ratio with respect to the β-hydroxy ester of 3.0 to 5.0, or any range of 0.5 therebetween. Preferably the ratio is between 3.5 and 4.0, more preferably 3.7.

The stoichiometric ratio of the ester starting material with respect to the β-hydroxy ester is preferably greater or equal to that of lithium with respect to the β-hydroxy ester.

The method may involve adding a conjugated diene as an electron transfer agent in a stoichiometric ratio with respect to the β-hydroxy ester of 1.0 to 2.5, or any range of 0.5 therebetween. Preferably the ratio is between 1.5 and 2.0, more preferably 1.8.

The stoichiometric ratio of the conjugated diene with respect to the β-hydroxy ester is preferably at least half that of lithium with respect to the β-hydroxy ester.

The reaction is preferably carried out as a batch process. Stirred tank reactors or vessels may also be used. Alternatively, at least one step may be carried out in a continuous manner.

### Examples

### Example 1

A crossed Claisen condensation reaction according to the present invention between tert-butyl acetate and HN, in order to produce ATS-8, was carried out as outlined below.

Lithium metal (3.52g, rods ∼ 5mm x 15mm) was charged to an argon inerted vessel. Tert-butyl methyl ether (TBME, 63g; 85ml) was added and the mixture stirred and cooled to -75°C.

Liquid ammonia (70ml) was charged to a nitrogen inerted measuring cylinder cooled to -75°C. The liquid ammonia was added in one portion to the TBME / lithium slurry. The reaction mixture turned dark blue after 2-3 minutes and then turned bronze / brown. An exotherm to -50°C was noted.

The mixture was stirred for 10 minutes and then styrene (25.44g; 28ml) was charged via a syringe pump over 30 minutes. During addition the temperature was maintained between -58 and -50°C.

After stirring for 10 minutes, tert-butyl acetate (TBA, 58.9g; 68ml) was added over 10 minutes, maintaining the temperature at -62 to -52°C. At the end of this addition, the mixture was stirred for 15 minutes, maintaining the temperature at -50 to -46°C. At the end of the stirring a pale, slightly hazy solution had formed.

(R)-ethyl-4-cyano-3-hydroxybutyrate (HN, 21.3g; 19ml) was then charged via a syringe pump, maintaining the temperature at -43 to -46°C to give clear yellow solution. After stirring for 2.5 hours at -40 to -45°C, the solution was warmed to 20°C to give a viscous dark brown solution. Frothing was observed as the ammonia evaporated off.

The reaction mixture was quenched into pre-cooled (1°C) 14% HCl over 20 minutes, keeping the temperature below 15°C. The final pH was 0.1. The reaction mixture was then allowed to settle overnight.

The lower pale yellow aqueous phase was separated off and the organic layer charged to a RFE flask. The organic layer was concentrated under vacuum, maintaining the temperature at 40 to 45°C, to give an orange / brown oil (47.9g). Heptane (43.4g; 63ml) was then added and the mixture stirred for 43 minutes. The heptane layer was then separated off. The remaining product layer was washed with further heptane (43.4g; 63ml) and separated off.

The product layer was concentrated under vacuum at 40 to 45°C to yield 27.1g of an orange oil. The product layer by HPLC was as follows; product = 96.41%; tert-butyl acetoacetate (TBAA, self condensation product) =2.95%; (R)-ethyl-4-cyano-3-hydroxybutyrate = 0.39%; total other impurities = 0.25%. The molar yield based on the HPLC assay was therefore 92%.

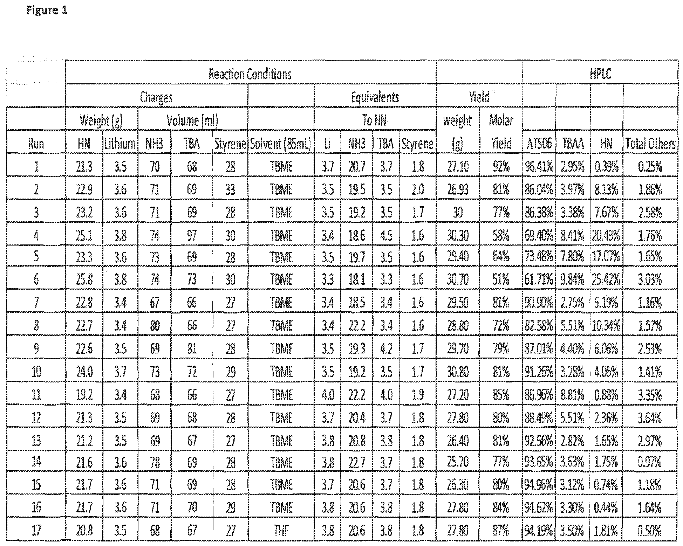

The results of various repeats following the above procedure but with varying reaction conditions are shown in Figure 1.

### Example 2

Calorimetry experiments were also run, using TBME as a solvent and styrene as an electron transfer agent. The experimental procedure is outlined below.

A 500ml power compensation calorimeter reactor was fitted with agitator, calibration heater, thermocouple, jacketed addition funnel (fitted with the dose temperature probe) and wet gas meter. TBME (153g) was charged under nitrogen and cooled to -49°C (10°C differential between the jacket and contents temperature) and stirred until steady state had been achieved. Liquid ammonia (85.7g) was charged to the addition funnel. The nitrogen purge was isolated and the liquid ammonia added in small portions to the TBME. At the end of the addition, the reaction mixture was again stirred until steady state conditions had been achieved.

Lithium metal (6.35g, rods ∼ 5mm x 15mm) was added one piece at a time. Once the exotherm had subsided, the next piece was added. After approximately half of the lithium had been added, the addition rate was increased to two pieces of lithium per addition. Once the addition had finished, the reaction mix was stirred to achieve steady state conditions at -49°C. The addition was carried out over approximately 2.5 hours.

Styrene (45.74g) was then added, initially targeted at 0.4g / min and then slowed to 0.3g / min, giving a total addition time of 2 hours. At the end of the addition, the reaction mix was stirred for approximately 1 hour prior to starting the TBA addition.

TBA (106.3g) was then added (at -49°C) at 3.5 g/min. At the start of this addition the energy output was too great and the power input by the coil fell to 0W. The addition rate was then reduced slowly and finally reduced to 2 g/min. The total addition time was 45 minutes. After stirring for approximately 50 minutes, the reactor was warmed to -43°C and allowed to equilibrate.

HN (38.35g) was then added via a syringe pump over 35 minutes, maintaining the temperature at - 43°C. The reaction mixture was then stirred for 3.5 hours prior to warming to evaporate the ammonia.

Evaporation was carried out by applying a 5°C temperature differential between the oil inlet temperature and the reactor contents temperature. This required approximately 4 hours to raise the reactor temperature to 20°C. The total gas volume recorded during the entire run was 80.6 L. Of this amount, 77 L was evolved during the final warm to room temperature. At this reaction scale, assuming a gas temperature of 0°C at the gas meter (and ideal behaviour), a total of 113 L would be expected - i.e. 72% of the theoretical off gas has been accounted for. The initial typical gas flow was 500ml / min, falling to 300 ml / min above a reactor temperature of -22°C. This equates to 13 and 8 L / min respectively gas flow for a 1kg scale HN reaction. The gaseous ammonia evaporated from the system can be compressed back into the liquid state and reused in the process if desired.

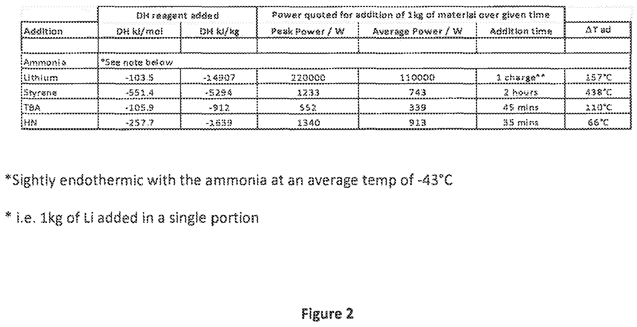

The results are shown in Figure 2.

### Example 3

The process of the present invention was scaled up, using TBME as a solvent and isoprene as an electron transfer agent. The scale up was done as outlined below.

A 1000L reactor was charged with TBME (200L). Lithium metal pellets (11.1kg) were added and the mixture cooled to -60°C over 45 minutes. Liquid ammonia (132kg) was added at a rate sufficient to maintain the temperature between -40 and -60°C. The addition took 6 hours. A solution of isoprene (52kg) in TBME (25L) was added over 7.17 hours at -48 to -56°C and the mixture stirred for 1 hour. TBA (172kg) was added over 1.5 hours at -45 to -58°C and the mixture stirred for 1 hour.

HN (50kg) was added over 1.5 hours at -43 to -46°C and the mixture stirred for 1 hour.

Methanol (50L, added to destroy any excess lithium) was added over 45 minutes at -35 to -45°C.

A solution of 20% sulphuric acid was prepared from concentrated sulphuric acid (300kg) and water (1200L) in a 5000L reactor and cooled to 7°C. 9L of this solution was removed for later pH adjustment.

The reaction mass was transferred into the sulphuric acid solution at 5 to 16°C over 3 hours. The pH was adjusted to between 6.0 and 6.5 by adding back some of the retained sulphuric acid solution. The mixture was stirred for 2 hours then settled for 30 minutes.

The aqueous layer was extracted with 2 x 100L of TBA and the organic layers combined.

The organic layers were evaporated under vacuum to a maximum temperature of 45°C. The residue was washed with hexane (2 x 150L) and then evaporated under vacuum at 45°C for 4 hours to afford 61.0kg of brown oil product. The product layer by HPLC was as follows; product = 89.2%; tert-butyl acetoacetate (TBAA, self condensation product) =6.73%; (R)-ethyl-4-cyano-3-hydroxybutyrate = 0.88%; total other impurities = 3.2%. The molar yield based on the HPLC assay was therefore 75%.

### Comparative Example 1

The inventors carried out the following reaction to demonstrate that the use of lithium amide in the absence of liquid ammonia does not produce the desired Claisen condensation reaction products.

A 250ml 3-necked vessel was equipped with an agitator, cooling bath and thermometer. The vessel was inerted using nitrogen and lithium amide (7.13g, 0.31 moles, purchased from Chemetall) was added. Tert-butyl-methyl ether (TBME, 50ml) was added and the white slurry cooled to -50°C.

Tert-butyl acetate (TBA, 36.07g, 0.31 moles) was then added in one portion. The mixture was stirred at -45°C for 1 hour after which time there was no change in appearance and no evidence of enolate formation (Note: the lithium enolate of TBA is completely soluble in TBME, so if the reaction progressed all solids would dissolve).

The mixture was warmed to 20°C and stirred at this temperature for 17.5 hours. After this time there was no change in the appearance of the mixture which remained a white slurry. Sampling revealed no evidence of self-condensation, further indicating the TBA-enolate had not formed.

It can be concluded from this experiment that in the absence of liquid ammonia lithium amide is not sufficiently soluble to enable the desired reaction to occur.

## Claims

1. Use of lithium amide in liquid ammonia as a base to produce an enolate from at least one ester starting material in a crossed Claisen condensation reaction, wherein at least one ester starting material is a β-hydroxy ester.

2. Use according to Claim 1, wherein:
i) two ester starting materials are used in the crossed Claisen condensation reaction, both of which are enolizable; and/or
ii) one ester starting material is enolizable and the other is a β-hydroxy ester, which may also be enolizable.

3. Use according to any preceding claim, wherein:
i) the lithium amide is produced *in situ*; preferably wherein
ii) the lithium amide is produced from lithium metal, liquid ammonia and an electron transfer agent, optionally selected from styrene or isoprene.

4. Use according to any preceding claim, wherein a co-solvent is used, wherein optionally:
the co-solvent is an ethereal solvent, a hydrocarbon solvent or a hindered ethereal solvent; preferably wherein
the co-solvent is selected from tert-butyl methyl ether, THF or hexane.

5. Use according to any preceding claim, wherein:
ammonia can be driven off the product on heating, and can then optionally be recompressed and recycled.

6. Use according to any preceding claim, wherein the lithium salt is quenched with one or more of an aqueous mineral acid, a carboxylic acid or an acidic ammonium salt; preferably wherein
i) the aqueous mineral acid is selected from hydrochloric acid and sulphuric acid; or
ii) the carboxylic acid is acetic acid; or
iii) the acidic ammonium salt is selected from ammonium chloride and ammonium nitrate.

7. Use according to any preceding claim, wherein the enolisable ester starting material is tert-butyl acetate, optionally wherein:
i) the β-hydroxy ester is HN, preferably the reaction product is 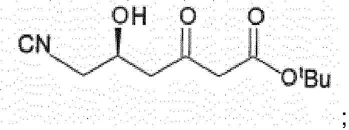 or
ii) the β-hydroxy ester is ECHB, preferably the reaction product is 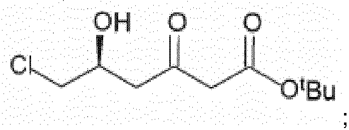 or
iii) the β-hydroxy ester is (S)-3-hydroxy-butyrolactone, preferably the reaction product is (S)-tert-butyl 5,6-dihydroxy-3-oxohexanoate.

8. A method of carrying out a crossed Claisen condensation reaction using an ester starting material and a β-hydroxy ester, comprising the step of producing lithium amide *in situ,* wherein lithium is added to liquid ammonia, followed by an electron transfer agent, wherein: i) lithium is added in a stoichiometric ratio with respect to the β-hydroxy ester of 3.0 to 5.0; and/or ii) ammonia is added in a stoichiometric ratio with respect to β-hydroxy ester of 5 to 30.

9. The method according to Claim 8, wherein the reaction temperature at ambient pressure for the production of the lithium amide *in situ* is maintained between -33 and -40°C, between -40 and -50°C, between -50 and -60°C or between -60 and -70°C.

10. The method according to Claim 8, wherein:
i) the production of the lithium amide *in situ* is carried out under increased pressure; preferably wherein
ii) the pressure and temperature for the production of the lithium amide *in situ* are balanced such that the ammonia exists in its liquid state.

11. The method according to Claim 8, wherein a conjugated diene is the electron transfer agent and is added in a stoichiometric ratio with respect to the β-hydroxy ester of 1.0 to 2.5.

12. The method according to Claim 11, wherein the stoichiometric ratio of the conjugated diene with respect to the β-hydroxy ester is at least half that of lithium with respect to the β-hydroxy ester.

13. The method according to any one of Claims 8 to 12, wherein:
an ester starting material is added in a stoichiometric ratio with respect to the β-hydroxy ester of 3.0 to 5.0; and/or
the stoichiometric ratio of the ester starting material with respect to the β-hydroxy ester is greater or equal to that of lithium with respect to the β-hydroxy ester.

## Patentansprüche

1. Verwendung von Lithiumamid in flüssigem Ammoniak als eine Basis zur Herstellung eines Enolats aus einem Ester-Ausgangsmaterial in einer gekreuzten Claisen-Kondensationsreaktion, wobei zumindest ein Ester-Ausgangsmaterial ein β-Hydroxy-Ester ist.

2. Verwendung nach Anspruch 1, wobei:
i) zwei Ester-Ausgangsmaterialien in der gekreuzten Claisen-Kondensationsreaktion verwendet werden, wobei beide enolisierbar sind; und/oder
ii) ein Ester-Ausgangsmaterial enolisierbar ist und das andere ein β-Hydroxy-Ester, der ebenfalls enolisierbar sein kann.

3. Verwendung nach einem vorhergehenden Anspruch, wobei:
i) das Lithiumamid in situ hergestellt wird; wobei vorzugsweise
ii) das Lithiumamid aus Lithium-Metall, flüssigem Ammoniak und einem Elektronentransfer-Mittel, optional ausgewählt aus Styrol und Isopren hergestellt wird.

4. Verwendung nach einem vorhergehenden Anspruch, wobei ein Cosolvens verwendet wird, wobei optional:
das Cosolvens ein etherisches Lösungsmittel, ein Kohlenwasserstoff-Lösungsmittel oder ein gehindertes etherisches Lösungsmittel ist; wobei vorzugsweise das Cosolvens aus tert-Butylmethylether, THF oder Hexan ausgewählt ist.

5. Verwendung nach einem vorhergehenden Anspruch, wobei:
Ammoniak durch Erwärmung von dem Produkt abgestoßen werden kann, und dann optional wieder verdichtet und zurückgeführt werden kann.

6. Verwendung nach einem vorhergehenden Anspruch, wobei das Lithiumsalz mit einem oder mehren einer wässrigen Mineralsäure, einer Carbonsäure oder einem sauren Ammoniumsalz abgeschreckt wird; wobei vorzugsweise
i) die wässrige Mineralsäure ausgewählt ist aus Salzsäure und Schwefelsäure; oder
ii) die Carbonsäure Essigsäure ist; oder
iii) das saure Ammoniumsalz aus Ammoniumchlorid und Ammoniumnitrat ausgewählt ist.

7. Verwendung nach einem vorhergehenden Anspruch, wobei das enolisierbare Ester-Ausgangsmaterial tert-Butylacetat ist, wobei optional:
i) der β-Hydroxy-Ester HN ist, wobei vorzugsweise das Reaktionsprodukt 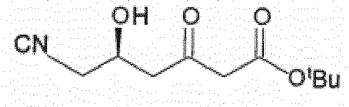 ist; oder
ii) der β-Hydroxy-Ester ECHB ist, vorzugsweise das Reaktionsprodukt 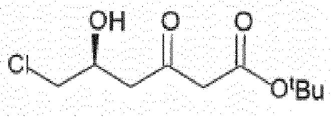 ist; oder
iii) der β-Hydroxy-Ester (S)-3-Hydroxy-butyrolacton ist, wobei vorzugsweise das Reaktionsprodukt (S)-tert-Butyl-5,6-dihydroxy-3-oxohexanoat ist.

8. Verfahren zur Durchführung einer gekreuzten Claisen-Kondensationsreaktion unter Verwendung eines Ester-Ausgangsmaterials und eines β-Hydroxy-Esters, umfassend den Schritt der Herstellung von Lithiumamid in situ, wobei Lithium zu flüssigem Ammoniak zugegeben wird, gefolgt von einem Elektronentransfer-Mittels, wobei: i) Lithium in einem stöchiometrischen Verhältnis in Bezug auf den β-Hydroxy-Ester von 3,0 bis 5,0 zugegeben wird; und/oder ii) Ammoniak in einem stöchiometrischen Verhältnis in Bezug auf den β-Hydroxy-Ester von 5 bis 30 zugegeben wird.

9. Verfahren nach Anspruch 8, wobei die Reaktionstemperatur bei Umgebungsdruck für die Herstellung des Lithiumamids in situ zwischen -33 und -40 °C, zwischen -40 und -50 °C, zwischen -50 und -60 °C, oder zwischen -60 und -70 °C gehalten wird.

10. Verfahren nach Anspruch 8, wobei:
i) die Herstellung des Lithiumamids in situ unter erhöhtem Druck erfolgt; wobei vorzugsweise
ii) der Druck und die Temperatur für die Herstellung des Lithiumamids in situ so ausgeglichen werden, dass das Ammoniak in seinem flüssigen Zustand vorliegt.

11. Verfahren nach Anspruch 8, wobei ein konjugiertes Dien das Elektronentransfer-Mittel ist und in einem stöchiometrischen Verhältnis in Bezug auf den β-Hydroxy-Ester von 1,0 bis 2,5 zugegeben wird.

12. Verfahren nach Anspruch 11, wobei das stöchiometrische Verhältnis des konjugierten Diens in Bezug auf den β-Hydroxy-Ester zumindest die Hälfte des Verhältnisses von Lithium in Bezug auf den β-Hydroxy-Ester ist.

13. Verfahren nach einem der Ansprüche 8 bis 12, wobei:
ein Ester-Ausgangsmaterial in einem stöchiometrischen Verhältnis in Bezug auf den β-Hydroxy-Ester von 3,0 bis 5,0 zugegeben wird; und/oder
das stöchiometrische Verhältnis des Ester-Ausgangsmaterials in Bezug auf den β-Hydroxy-Ester größer oder gleich jenem von Lithium in Bezug auf den β-Hydroxy-Ester ist.

## Revendications

1. Utilisation d'amide de lithium dans de l'ammoniac liquide en tant que base pour produire un énolate à partir d'au moins un matériau de départ à base d'ester dans une réaction de condensation croisée de Claisen, où au moins un matériau de départ à base d'ester est un β-hydroxy-ester.

2. Utilisation selon la revendication 1, dans laquelle :
i) deux matériaux de départ à base d'ester sont utilisés dans la réaction de condensation croisée de Claisen, tous deux étant énolisables ; et/ou
ii) un matériau de départ à base d'ester est énolisable et l'autre est un β-hydroxy-ester, qui peut également être énolisable.

3. Utilisation selon l'une des revendications précédentes, dans laquelle :
i) l'amide de lithium est produit *in situ,* de préférence dans laquelle
ii) l'amide de lithium est produit à partir de lithium métallique, d'ammoniac liquide et d'un agent de transfert d'électrons, facultativement choisi parmi le styrène ou l'isoprène.

4. Utilisation selon l'une des revendications précédentes, dans laquelle un co-solvant est utilisé, dans laquelle facultativement :
le co-solvant est un solvant éthéré, un solvant hydrocarboné ou un solvant éthéré encombré ; de préférence dans laquelle
le co-solvant est choisi parmi le tert-butyl méthyl éther, le THF ou l'hexane.

5. Utilisation selon l'une des revendications précédentes, dans laquelle :
l'ammoniac peut être chassé du produit lors du chauffage, et peut ensuite facultativement être recomprimé et recyclé.

6. Utilisation selon l'une des revendications précédentes, dans laquelle le sel de lithium est trempé avec un ou plusieurs parmi un acide minéral aqueux, un acide carboxylique ou un sel d'ammonium acide ; de préférence dans laquelle
i) l'acide minéral aqueux est choisi parmi l'acide chlorhydrique et l'acide sulfurique ; ou
ii) l'acide carboxylique est l'acide acétique ; ou
iii) le sel d'ammonium acide est choisi parmi le chlorure d'ammonium et le nitrate d'ammonium.

7. Utilisation selon l'une des revendications précédentes, dans laquelle le matériau de départ à base d'ester énolisable est l'acétate de tert-butyle, facultativement dans laquelle :
i) le β-hydroxy-ester est HN, de préférence le produit de réaction est 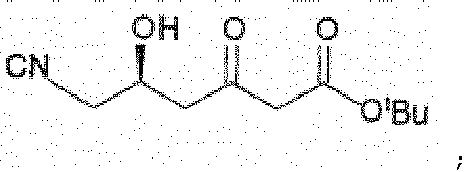 ou
ii) le β-hydroxy-ester est ECHB, de préférence le produit de réaction est 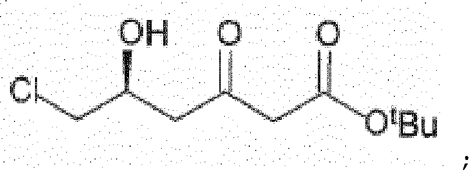 ou
iii) le β-hydroxy-ester est le (S)-3-hydroxy-butyrolactone, de préférence le produit de réaction est le (S)-5,6-dihydroxy-3-oxohexanoate de tert-butyle.

8. Procédé de réalisation d'une réaction de condensation croisée de Claisen en utilisant un matériau de départ à base d'ester et un β-hydroxy-ester, comprenant l'étape de production *in situ* d'amide de lithium, où du lithium est ajouté à de l'ammoniac liquide, suivi d'un agent de transfert d'électrons, où : i) du lithium est ajouté dans un rapport stœchiométrique par rapport au β-hydroxy-ester de 3,0 à 5,0 ; et/ou ii) de l'ammoniac est ajouté dans un rapport stœchiométrique par rapport au β-hydroxy-ester de 5 à 30.

9. Procédé selon la revendication 8, dans lequel la température de réaction à la pression ambiante pour la production de l'amide de lithium *in situ* est maintenue entre -33 et -40°C, entre -40 et -50°C, entre -50 et -60°C ou entre -60 et -70°C.

10. Procédé selon la revendication 8, dans lequel :
i) la production de l'amide de lithium *in situ* est réalisée sous une pression accrue ; de préférence dans lequel
ii) la pression et la température pour la production de l'amide de lithium *in situ* sont équilibrées de sorte que l'ammoniac existe dans son état liquide.

11. Procédé selon la revendication 8, dans lequel un diène conjugué est l'agent de transfert d'électrons et est ajouté dans un rapport stœchiométrique par rapport au β-hydroxy-ester de 1,0 à 2,5.

12. Procédé selon la revendication 11, dans lequel le rapport stœchiométrique du diène conjugué par rapport au β-hydroxy-ester est au moins égal à la moitié de celui du lithium par rapport au β-hydroxy-ester.

13. Procédé selon l'une quelconque des revendications 8 à 12, dans lequel :
un matériau de départ à base d'ester est ajouté dans un rapport stœchiométrique par rapport au β-hydroxy-ester de 3,0 à 5,0 ; et/ou
le rapport stœchiométrique du matériau de départ à base d'ester par rapport au β-hydroxy-ester est supérieur ou égal à celui du lithium par rapport au β-hydroxy-ester.
